# FASCICULE DE BREVET EUROPEEN

(11) **EP 1 517 721 B1**
(45) Date de publication et mention de la délivrance du brevet: **22.10.2008**
(21) Numéro de dépôt: 03729790.0
(22) Date de dépôt: 02.07.2003
(51) Int. Cl.: A61M 37/00, A61M 5/50

(54) **DISPOSITIF POUR L'INSERTION D'IMPLANTS**
GERÄT ZUM EINFÜHREN VON IMPLANTATEN
IMPLANT INSERTING DEVICE

(30) Priorité: 03.07.2002 WO PCT/CH02/00360
(43) Date de publication de la demande: 30.03.2005
(73) Titulaire: DEBIOPHARM S.A., 1002 Lausanne (CH)
(72) Inventeur: MATHIEU, Christian, c/o AP TECHNOLOGIES SA, CH-1348 Le Brassus (CH); HEIMGARTNER, Frédéric, c/o DEBIO R.P., CH-1920 Martigny (CH); BESSEGHIR, Kamel, c/o DEBIOPHARM SA, CH-1000 Lausanne 9 (CH)
(74) Mandataire: Besse, François
(86) Numéro de dépôt international: PCT/CH2003/000437
(87) Numéro de publication internationale: WO 2004/004822

(56) Documents cités:
- DE-A- 4 106 196
- FR-A- 2 351 642
- FR-A- 2 355 491
- US-A- 4 223 674
- US-A- 4 576 591
- US-A- 4 673 387
- US-A- 6 102 844

## Description

L'invention concerne un dispositif pour l'insertion d'implants en forme de cylindre de diamètre étroit. Elle se rapporte plus particulièrement à un dispositif permettant d'insérer successivement plusieurs implants au niveau sous-cutané.

L'insertion successive d'implants trouve plusieurs applications dans le domaine médical, notamment dans le traitement de maladies chroniques telles que l'acromégalie, lorsque le patient reçoit une série d'implants le plus souvent biodégradables contenant un principe actif.

Le brevet américain US 4,086,914 décrit un dispositif pour l'insertion d'une série d'implants déposés successivement. Le dispositif comprend un trocart et un poussoir en forme de tige monté coulissant à travers le trocart. L'introduction des implants dans le dispositif s'effectue au moyen d'une cartouche de forme tubulaire qui contient une série d'implants. La cartouche est initialement introduite dans un compartiment situé dans le prolongement de l'extrémité proximale du trocart. Au moyen du poussoir, la série d'implants est ensuite déplacée en direction de l'extrémité distale du trocart.
Si, pour un traitement donné, le nombre d'implants contenus dans la cartouche est insuffisant, la cartouche vide est remplacée par une cartouche pleine.

Un autre exemple d'un dispositif pour l'insertion d'implants est décrit dans FR-A- 2 351 642.

Le remplacement d'une cartouche est une opération délicate. Plusieurs manipulations sont nécessaires, ce qui non seulement résulte en une augmentation de la durée du traitement, mais également en des douleurs supplémentaires pour le patient qui sont dues au mouvement du trocart lors du remplacement de cartouche.

La présente invention comme revendiquée vise notamment à remédier aux inconvénients précités.

Elle concerne un dispositif, pour l'insertion d'implants, en forme de cylindre de diamètre étroit comprenant des moyens de saisie, une aiguille creuse (nommée trocart par la suite) fixée par son extrémité proximale aux moyens de saisie, et un poussoir en forme de tige monté coulissant à travers le trocart et les moyens de saisie, caractérisé en ce que les moyens de saisie comportent un élément rotatif définissant un axe de rotation parallèle à l'axe du trocart et comprenant une pluralité d'éléments tubulaires disposés autour dudit axe de rotation et montés de manière à pouvoir s'aligner successivement avec le trocart, ledit élément rotatif faisant partie intégrante des moyens de saisie et s'étendant sur la plus grande partie de leur longueur, chaque élément tubulaire étant adapté pour contenir au moins un implant.

Les éléments tubulaires peuvent être formés directement dans l'élément rotatif.
Pour des raisons d'exigences réglementaires liées aux instruments médicaux, notamment pour des raisons de stérilité du dispositif, mais également afin d'assurer une conservation optimale des implants, il est toutefois souhaitable d'utiliser des éléments tubulaires qui forment une partie distincte de l'élément rotatif. Cette configuration offre la possibilité de séparer les implants du dispositif d'insertion préalablement au traitement.
En outre, si le traitement ne nécessite pas l'utilisation de toutes les cartouches qui peuvent être chargées sur l'élément rotatif, il est possible de ne charger que le nombre nécessaire de cartouches. Ceci permet d'assurer au patient l'administration de la dose exacte pour le traitement de sa maladie. Par ailleurs, dans bien des cas, l'économie ainsi réalisée peut être très importante, le prix d'un implant pouvant être très élevé.
A noter que le chargement de l'élément rotatif peut être effectué à l'usine ou par le praticien, préalablement au traitement.

Un exemple de réalisation de l'invention sera décrit ci-après au moyen des figures suivantes :
La figure 1 représente le dispositif d'insertion prêt à l'emploi
La figure 2 montre une vue frontale du dispositif
La figure 3 montre une vue éclatée du dispositif
La figure 4 illustre le barillet et quelques cartouches
La figure 5 montre une cartouche contenant une série de cinq implants

Liste références numériques utilisées sur les figures :
1. Dispositif d'insertion d'implants
2. Elément de saisie
3. Trocart
4. Extrémité proximale du trocart
5. Poussoir
6. Barillet
7. Axe de rotation du barillet
8. Axe principal du trocart
9. Cartouche
10. Implant
11. Languette de retenue
12. Clip de retenue distale
13. Gorge
14. Fenêtre
15. Section transversale de l'élément de saisie
16. Molette
17. Clip de retenue proximale
18. Compartiment pour cartouche

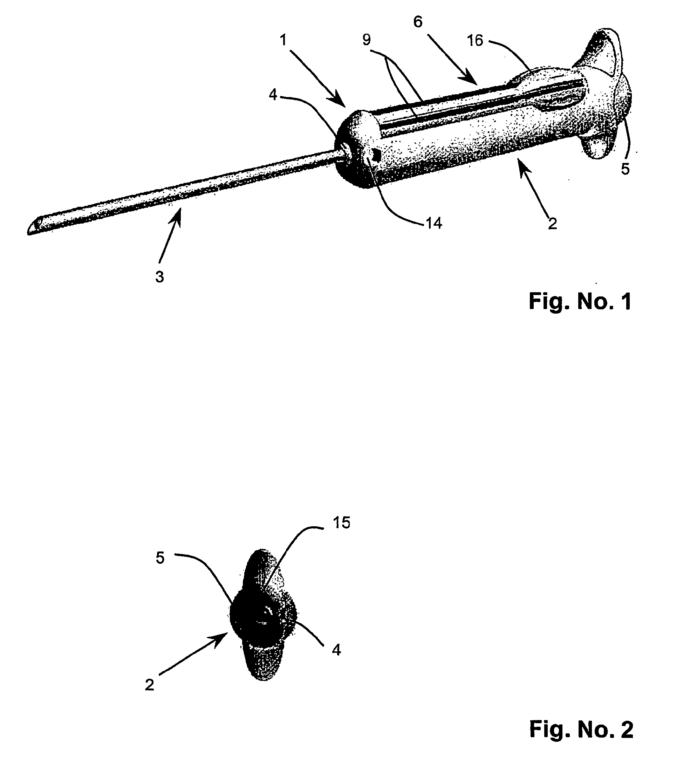

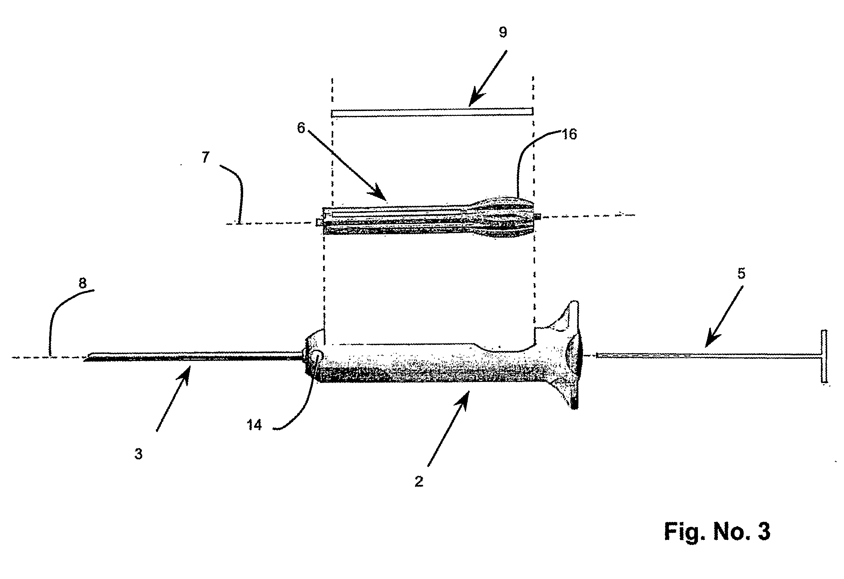

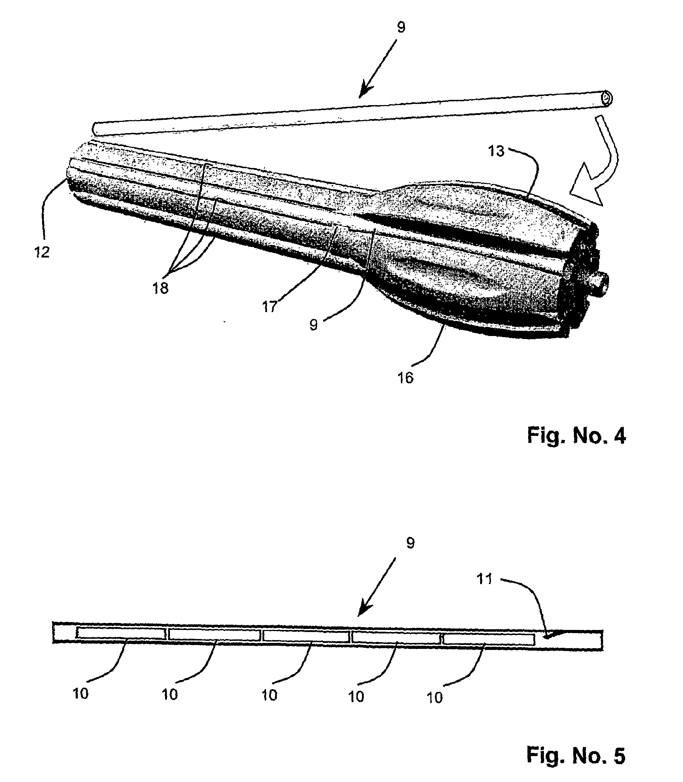

Le dispositif illustré sur les figures 1 et 3 comprend un trocart **3,** un élément de saisie **2** sur lequel est fixée l'extrémité proximale **4** du trocart **3.** Un poussoir **5** en forme de tige est monté coulissant à travers le trocart **3** et l'élément de saisie **2.** Le diamètre externe de la tige du poussoir **5** est sensiblement égal au diamètre interne du trocart 3.
L'élément de saisie **2** comprend un élément rotatif **6,** nommé barillet par la suite (voir également la figure 4), muni à son extrémité proximale d'un renflement formant une molette **16.** Le barillet **6** comporte plusieurs éléments tubulaires **9,** en métal par exemple, nommés cartouches par la suite, qui sont fixés par encliquetage sur le corps du barillet **6.** Selon un mode préférentiel, cinq cartouches **9** sont utilisées. Chaque cartouche **9** contient une série d'implants **10** disposés successivement l'un derrière l'autre. Le choix des dimensions des implants n'est pas limité. Cependant, on choisira en général une longueur se situant entre 0.5 et 2 cm et un diamètre de 1.7 à 1.9 mm. L'axe de rotation **7** du barillet **6** est parallèle à l'axe principal du dispositif **1,** notamment à l'axe **8** du trocart **3.** Le barillet **6** est disposé de manière à autoriser l'alignement successif des cartouches **9** avec le trocart **3.** L'élément de saisie **2** comporte en outre une fenêtre **14** permettant de visualiser le passage des implants **10** lors de leur déplacement à travers l'élément de saisie **2.** A la place de la fenêtre **14,** on peut prévoir un autre moyen de détermination de la position du poussoir **5,** par exemple une graduation disposée sur le poussoir **5.**
Comme on peut le remarquer en particulier à la figure 4, le barillet **6** comporte une série de compartiments **18** dans lesquels viennent se loger les cartouches **9.** Une fois logées, ces dernières ne peuvent être retirées à cause de la présence de clips de retenue **12,17** et des gorges **13** de la molette **16** au fond desquelles se logent les cartouches **9.** L'usage unique des cartouches **9** est ainsi assuré.
L'élément de saisie **2** est de préférence conçu pour que le barillet **6** soit ensuite introduit dans l'élément de saisie **2** au niveau de son extrémité distale.
La figure 5 illustre une cartouche **9** contenant une série de cinq implants **10** disposés successivement. Lorsque le dispositif **1** est au repos, le déplacement vers l'arrière des implants **10** est empêché par la présence d'une languette flexible **11** située vers l'extrémité proximale de la cartouche **9.** En outre, un système similaire de retenue des implants **10** peut être disposé vers l'extrémité distale de la cartouche **9.**
La section **15** de l'élément de saisie **2** est de forme ovale ou plus généralement aplatie (voir figure 2) afin de faciliter le positionnement du dispositif sur la peau du patient et de réduire la douleur qui pourrait être induite par un trocart formant un angle important avec la surface de la peau.

Selon un mode préférentiel non illustré, le dispositif comprend des moyens de retenue du barillet **6** qui empêchent le retrait du barillet une fois celui-ci mis en place sur l'élément de saisie **2.** Ces moyens peuvent être constitués d'une languette de retenue qui se brise si l'on cherche à retirer le barillet.

Le fonctionnement du dispositif sera décrit ci-après.
De préférence, préalablement à son utilisation, le dispositif est séparé en différentes parties. Les cartouches **9** contenant les implants **10** sont conservées à part, le barillet **6** n'est pas fixé à l'élément de saisie **2.**
Afin de compacter l'ensemble, le poussoir **15** peut être complètement introduit dans l'élément de saisie **2** et dans le trocart **3.**
Dans une première étape, les cartouches **9** sont chargées sur le barillet **6** par encliquetage.
Le barillet **6** est ensuite fixé sur l'élément de saisie **2** comme indiqué plus haut.
Préalablement à la fixation du barillet **6** sur l'élément de saisie **2,** le poussoir **5** peut être retiré.
Alternativement, le poussoir **5** reste en place. Dans ce cas, le barillet **6** contient un compartiment (non-illustré) sans cartouche qui vient se positionner exactement sur la tige du poussoir **5.**
Une fois le barillet **6** mis en place, la pointe du trocart **3** est introduite sous la peau du patient. A ce moment, le poussoir **5** est de préférence enfoncé afin d'éviter un effet emporte-pièce.

Le poussoir **5** est alors retiré de manière à autoriser l'alignement avec le trocart **3** d'une première cartouche **9** pleine par rotation du barillet **6** au niveau de la molette **16.**
On relèvera ici que de préférence, la rotation du barillet s'effectue de manière discontinue, par incrément (encliquetage), de façon à aligner immédiatement les cartouches **9** avec le trocart **3.**
Le poussoir **5** est alors déplacé en direction de l'extrémité distale du trocart **3.** Se faisant, la languette flexible **11** est rabattue vers l'extérieur de la cartouche **9.**

Lorsque les implants **10** se trouvent vers l'extrémité distale du trocart **3,** l'élément de saisie **2** est progressivement retiré tandis que le poussoir **5** reste en position fixe afin de déposer le chapelet d'implants **10** dans la cavité ménagée par le trocart **3** sous la peau.

Une fois la cartouche **9** vidée, cette phase étant constatée au travers de la fenêtre **14,** le poussoir **5** est retiré de manière à autoriser une nouvelle rotation du barillet **6** pour aligner une nouvelle cartouche **9** avec le trocart **3.** Le déroulement de la procédure étant identique avec les autres cartouches **9.**

## Revendications

1. Dispositif (1), pour l'insertion d'implants (10), en forme de cylindre de diamètre étroit comprenant des moyens de saisie (2), un trocart (3) fixé par son extrémité proximale (4) aux moyens de saisie (2), et un poussoir (5) en forme de tige monté coulissant à travers le trocart (3) et les moyens de saisie (2), **caractérisé en ce que** les moyens de saisie (2) comportent un élément rotatif (6) définissant un axe de rotation (7) parallèle à l'axe du trocart (8) et comprenant une pluralité d'éléments tubulaires (9) disposés autour dudit axe de rotation (7) et montés de manière à pouvoir s'aligner successivement avec le trocart (3), ledit élément rotatif (6) faisant partie intégrante des moyens de saisie (2) et s'étendant sur la plus grande partie de leur longueur, chaque élément tubulaire (9) étant adapté pour contenir au moins un implant.

2. Dispositif selon la revendication 1 **caractérisé en ce que** ce que chaque élément tubulaire (9) forme une partie distincte du reste de l'élément rotatif (6).

3. Dispositif selon la revendication 2 **caractérisé en ce que** chaque élément tubulaire (9) est insérable dans l'élément rotatif (6).

4. Dispositif selon la revendication 2 ou 3 **caractérisé en ce qu'**il comprend des moyens (12,13) qui empêchent le retrait des éléments tubulaires (9) de l'élément rotatif (6).

5. Dispositif selon l'une quelconque des revendications précédentes **caractérisé en ce qu'**il comporte des moyens pour visualiser le passage des implants qui se trouvent dans l'élément tubulaire (9) qui est aligné avec le trocart (3).

6. Dispositif selon la revendication précédente **caractérisé en ce que** les moyens pour visualiser le passage des implants comprennent une fenêtre (14).

7. Dispositif selon l'une quelconque des revendications précédentes **caractérisé en ce que** les moyens de saisie (2) ont une section (15) aplatie.

8. Dispositif selon l'une quelconque des revendications précédentes **caractérisé en ce que** l'élément rotatif (6) comprend une molette (16).

9. Dispositif selon l'une quelconque des revendications précédentes **caractérisé en ce que** chaque élément tubulaire (9) comporte des moyens (11) pour retenir les implants (10) lorsque le dispositif (1) est au repos.

10. Dispositif selon la revendication précédente **caractérisé par le fait que** les moyens pour retenir les implants sont constitués d'une languette flexible (11) disposée à l'intérieur des éléments tubulaires (9).

11. Dispositif selon l'une quelconque des revendications précédentes **caractérisé par le fait qu'**il comprend des moyens de retenue de l'élément rotatif (6) qui empêchent le retrait de l'élément rotatif (6) une fois celui-ci mis en place dans les moyens de saisie (2).

## Claims

1. Device (1) for inserting implants (10), which is in the form of a cylinder of small diameter and comprises gripping means (2), a trocar (3) fixed at its proximal end (4) to the gripping means (2), and a push rod (5) mounted so as to slide through the trocar (3) and the gripping means (2), **characterized in that** the gripping means (2) include a rotary element (6) defining an axis of rotation (7) parallel to the trocar axis (8) and comprising a plurality of tubular elements (9) arranged around said axis of rotation (7) and mounted so as to be able to be aligned successively with the trocar (3), said rotary element (6) forming an integral part of the gripping means (2) and extending along most of the length of said gripping means, each tubular element (9) being designed to contain at least one implant.

2. Device according to Claim 1, **characterized in that** each tubular element (9) forms a part distinct from the rest of the rotary element (6).

3. Device according to Claim 2, **characterized in that** each tubular element (9) can be inserted into the rotary element (6).

4. Device according to Claim 2 or 3, **characterized in that** it comprises means (12, 13) which prevent withdrawal of the tubular elements (9) from the rotary element (6).

5. Device according to any one of the preceding claims, **characterized in that** it comprises means for viewing the passage of the implants which are located in the tubular element (9) aligned with the trocar (3).

6. Device according to the preceding claim, **characterized in that** the means for viewing the passage of the implants comprise a window (14).

7. Device according to any one of the preceding claims, **characterized in that** the gripping means (2) have a flattened section (15).

8. Device according to any one of the preceding claims, **characterized in that** the rotary element (6) comprises a knurled wheel (16).

9. Device according to any one of the preceding claims, **characterized in that** each tubular element (9) includes means (11) for retaining the implants (10) when the device (1) is at rest.

10. Device according to the preceding claim, **characterized in that** the means for retaining the implants are composed of a flexible tongue (11) arranged inside the tubular elements (9).

11. Device according to any one of the preceding claims, **characterized in that** it comprises means which retain the rotary element (6) and prevent withdrawal of the rotary element (6) once the latter has been placed in the gripping means (2).

## Patentansprüche

1. Gerät (1) zum Einführen von Implantaten (10) in der Form von Zylindern mit kleinem Durchmesser, das Greifmittel (2), einen an seinem proximalen Ende (4) an den Greifmitteln (2) befestigten Trokar (3) und einen Schiebestab (5) aufweist, der so montiert ist, dass er durch den Trokar (3) und die Greifmittel (2) gleitet, **dadurch gekennzeichnet, dass** die Greifmittel (2) ein drehbares Element (6) aufweisen, das eine parallel zur Trokarachse (8) verlaufende Drehachse (7) definiert und mehrere rohrförmige Elemente (9) aufweist, die um die Drehachse (7) angeordnet und so montiert sind, dass sie nacheinander auf den Trokar (3) ausgerichtet werden können, wobei das drehbare Element (6) einen integralen Teil der Greifmittel (2) bildet und sich entlang dem größten Teil der Länge der Greifmittel erstreckt, wobei jedes rohrförmige Element (9) so ausgelegt ist, dass es mindestens ein Implantat enthält.

2. Gerät nach Anspruch 1, **dadurch gekennzeichnet, dass** jedes rohrförmige Element (9) ein vom restlichen drehbaren Element (6) getrenntes Teil bildet.

3. Gerät nach Anspruch 2, **dadurch gekennzeichnet, dass** jedes rohrförmige Element (9) in das drehbare Element (6) eingeführt werden kann.

4. Gerät nach Anspruch 2 oder 3, **dadurch gekennzeichnet, dass** es Mittel (12, 13) aufweist, die das Zurückziehen der rohrförmigen Elemente (9) aus dem drehbaren Element (6) verhindern.

5. Gerät nach einem der vorhergehenden Ansprüche, **dadurch gekennzeichnet, dass** es Mittel zum Einsehen des Durchgangs der Implantate aufweist, die sich in dem auf den Trokar (3) ausgerichteten rohrförmigen Element (9) befinden.

6. Gerät nach einem der vorhergehenden Ansprüche, **dadurch gekennzeichnet, dass** die Mittel zum Einsehen des Durchgangs der Implantate ein Fenster (14) umfassen.

7. Gerät nach einem der vorhergehenden Ansprüche, **dadurch gekennzeichnet, dass** die Greifmittel (2) einen abgeflachten Abschnitt (15) haben.

8. Gerät nach einem der vorhergehenden Ansprüche, **dadurch gekennzeichnet, dass** das drehbare Element (6) ein gerändeltes Rad (16) umfasst.

9. Gerät nach einem der vorhergehenden Ansprüche, **dadurch gekennzeichnet, dass** jedes rohrförmige Element (9) Mittel (11) zum Halten der Implantate (10), wenn das Gerät (1) ruht, aufweist.

10. Gerät nach einem der vorhergehenden Ansprüche, **dadurch gekennzeichnet, dass** die Mittel zum Halten der Implantate aus einer flexiblen Zunge (11) bestehen, die in den rohrförmigen Elementen (9) angeordnet ist.

11. Gerät nach einem der vorhergehenden Ansprüche, **dadurch gekennzeichnet, dass** es Mittel aufweist, die das drehbare Element (6) halten und das Zurückziehen des drehbaren Elements (6) verhindern, wenn dieses in die Greifmittel (2) platziert worden ist.
